Europäisches Patentamt

European Patent Office

Office européen des brevets

(11) Publication number: **0 289 952 B1**

## EUROPEAN PATENT SPECIFICATION

(45) Date of publication of patent specification: **01.07.92**   (51) Int. Cl.⁵: **C07C 309/00**, C07C 303/00

(21) Application number: **88106907.4**

(22) Date of filing: **29.04.88**

(54) Preparation of propanone -1,3-disulfonic acid.

(30) Priority: **01.05.87 US 44933**

(43) Date of publication of application:
**09.11.88 Bulletin 88/45**

(45) Publication of the grant of the patent:
**01.07.92 Bulletin 92/27**

(84) Designated Contracting States:
**AT BE CH DE ES FR GB GR IT LI LU NL SE**

(56) References cited:
**DE-B- 1 618 882**
**DE-C- 648 237**

**CHEMICAL ABSTRACTS, vol. 48, no. 12, June 25, 1954, Columbus, Ohio, US; A.P. TEREN-TEV et al.: "Sulfonation and sulfonic acids of acidophobic substances. XXIII. Sulfonation of aldehydes and ketones."**

**BERICHTE DER DEUTSCHEN CHEMISCHEN GESELLSCHAFT, edition 59, August 1926, vol. II, sep. B, Verlag Chemie, Berlin; F. RASCHING et al.: "Ueber das angebliche Oxy-methansulfonsaure Kalium von Max Müller."**

**THE JOURNAL OF ORGANIC CHEMISTRY, vol.**

30, Feb. 1965, Easton, Pennsylvania; W.G. GROT: "Sulfonation of Acetone with jFuming Sulfuric Acid and Sjome Reactions of Propanone-1.3-di-sulfonic Acid.", pages 515 - 517

(73) Proprietor: **ATOCHEM NORTH AMERICA, INC. (a Pennsylvania corp.)**
**Three Parkway**
**Philadelphia Pennsylvania 19102(US)**

(72) Inventor: **Sandler, Stanley R.**
**221 Hemlock Lane**
**Springfield Pennsylvania 19064(US)**

(74) Representative: **Kraus, Walter, Dr. et al**
**Patentanwälte Kraus, Weisert & Partner**
**Thomas-Wimmer-Ring 15**
**W-8000 München 22(DE)**

EP 0 289 952 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is directed to methods for the preparation of propanone-1,3-disulfonic acid, also more commonly known as acetone disulfonic acid.

### Background of the Invention

Methods for preparation of propanone-1,3-disulfonic acid have been known for over thirty-five years, but these methods have a number of serious deficiencies.

An early reported method involved the reaction of acetone with dioxane-sulfur trioxide complex to yield acetone disulfonic acid, which was isolated as the barium salt, A.P. Terentev and L.A. Yanovskaya, Doklady Akad. Nauk. S.S.S.R., 75,235 (1950); Chem. Abstracts, 45,8445i (1951); Zh. Obshch. Khim., 23,618 (1953); Chem. Abstracts, 48,6958 (1954). This method has the disadvantage of requiring dioxane-sulfur trioxide, which is not commercially available on a large scale.

It was later reported that propanone-1,3-disulfonic acid could be prepared by the sulfonation of acetone with fuming sulfuric acid or oleum, W. Grot, J. Org. Chem., 30,515 (1965) and German Patent 70,392 issued June 28, 1968 to F. Wolf. This method has the disadvantage of requiring one to separate the product from a large excess of sulfuric acid. Another difficulty is the preparation of oleum from sulfuric acid and sulfur trioxide, which is difficult to handle due to its narrow liquid range (boiling point 44.8 degrees Centigrade, freezing point 17 degrees Centigrade), and trace amounts of water act as a polymerization catalyst for liquid sulfur trioxide, producing polymers having elevated boiling points. In addition, oleum is not stable, and the sulfur trioxide sometimes separates as a lower layer which is difficult to re-dissolve.

In the same reference cited above, Grot also made reference to another method (no citation given) for the preparation of propanone-1,3-disulfonic acid from 1,3-dichloroacetone and alkali sulfites. This method would have the disadvantage of requiring expensive 1,3-dichloroacetone and would give the disodium or dipotassium salts rather than the free acid. The disodium or dipotassium salts are rather difficult to convert to the free acid. The procedure used for preparing the dipotassium salt was described earlier by F. Raschig and W. Prahl, Ber. der deutch. chem. Ges., 59B,2025 (1926).

U.S. Pat. 3,454,628 of G. Schroder et al discloses a method somewhat related to the Grot method described above. In the method of this patent acetone was reacted with fuming sulfuric acid in the presence of ammonium sulfate at 85 to 90 degrees Centigrade to directly prepare the diammonium salt of propanone-1,3-disulfonic acid. The free acid was not isolated from the salt, and this process also suffers from the same disadvantages as described above for the Grot process.

### Brief Summary of the Invention

According to the present invention a relatively simple method is provided for preparing propanone-1,3-disulfonic acid by reacting chlorosulfonic acid with acetone. The reaction is preferably carried out in the presence of a solvent, such as methylene chloride, for the reactants, and the precipitated product may be dissolved in water and separated from the solvent layer. Prior to the separation, the reaction should be under substantially anhydrous conditions.

### Detailed Description of Preferred Embodiments

Propanone-1,3-disulfonic acid (acetone disulfonic acid) is prepared by reacting chlorosulfonic acid with acetone in the presence or absence of a solvent. The reaction may be represented by the following equation:

$$2ClSO_3H + (CH_3)_2C=O \rightarrow (CH_2SO_3H)_2C=O + 2HCl\uparrow$$

The reaction is exothermic and proceeds rapidly with only slight to moderate warming of the reaction mixture. The hydrogen chloride which is formed as a by-product is liberated during the reaction, and when its evolution ceases the reaction is complete.

The chlorosulfonic acid is preferably 99-100% grade, which is available commercially from several sources. The acetone is also available commercially and is preferably 100%, anhydrous grade. Since chlorosulfonic acid reacts vigorously with water, it is preferred that the reactants and the reaction conditions be substantially anhydrous. The primary impurities in the chlorosulfonic acid are hydrochloric and sulfuric acids, which form from contamination with water.

The reaction may be carried out neat or in the presence of a solvent for the reactants. The use of a solvent is preferred, since the neat reaction mixture is difficult to stir, and the product forms as a solid lump. The use of a solvent allows easier handling, better mixing and improves reaction rate.

The solvent may be virtually any inert solvent which readily dissolves both reactants. Examples of suitable solvents include methylene chloride, carbon tetrachloride, 1,1,1-trichloroethane (methylchloroform), chloroform, dioxane, acetonitrile, tetrahydrofuran, and ethyl ether. Other suitable solvents will be evident to those skilled in the art. A particularly preferred solvent is methylene chloride.

Although the order of addition of the reactants is not particularly critical, it has been found preferable to dissolve the acetone in the solvent and add the resulting solution slowly to the chlorosulfonic acid in the reaction vessel. For example, the acetone solution may be added over a period of about one hour. After warming of the mixture to accelerate the reaction, the solvent can be refluxed while the reaction proceeds for about three to four hours.

The reaction proceeds readily at substantially atmospheric pressure, preferably under a dry air atmosphere, with simple agitation (e.g., stirring). Noticeable precipitation occurs throughout the reaction, and the evolved hydrogen chloride gas may be captured by conventional means.

The reaction temperature of the process will generally be in the range of 4 to 60 degrees Centigrade, and preferably 4 to 40 degrees Centigrade, unless the reaction is carried out under pressure. The upper end of the temperature range is limited by the lowest boiling component of the reaction mixture. For example, where methylene chloride is the solvent, the reaction at atmospheric pressure would be carried out at its boiling point of 40 degrees Centigrade or below. Where a higher boiling point solvent such as carbon tetrachloride (boiling point 76 degrees Centigrade) is used, the boiling point of acetone (56 degrees Centigrade) becomes the temperature limiting factor.

The reactants are generally present in about stoichiometric amounts, or preferably with a slight stoichiometric excess of chlorosulfonic acid. In particular, the mole ratio of chlorosulfonic acid to acetone can range from 2:1 to 2.3:1. A ratio of slightly less than 2:1 may also be acceptable due to the volatility and potential loss of some of the acetone. It is preferred that the amount of solvent be kept to a minimum, consistent with optimum mixing, handling and reaction conditions.

The white solid precipitate of propanone-1,3-disulfonic acid may be recovered by filtration or by dissolving in water. However, the solid product is highly hygroscopic and difficult to dry, so that filtration is not a preferred recovery method. When water is added to the completed reaction mixture, the product is isolated as an aqueous solution (free of solvent) which requires less time than filtration. Depending on the concentration the aqueous solution of product may have a greater density than the methylene chloride solvent and may be easily separated from the methylene chloride solvent which contains any unreacted acetone and chlorosulfonic acid. The product can be conveniently sold either as an anhydrous solid or more preferably as a 70% aqueous solution which does not require the expense of drying.

Since chlorosulfonic acid has a boiling point of 151-152 degrees Centigrade, it is easier to handle than sulfur trioxide used in prior art processes. Moreover, propanone-1,3-disulfonic acid is readily separated from methylene chloride and similar solvents for the reactants, and the use of such a solvent allows one to isolate the product as an aqueous solution. The process of the invention produces yields in excess of 75% of the desired product.

The present invention will now be illustrated in more detail by reference to the following specific, non-limiting examples. In the examples proton ($H^1$nmr) and carbon ($C^{13}$nmr) nuclear magnetic resonance and elemental analysis were used to identify the propanone-1,3-disulfonic acid. The solvents for the nmr analysis were deuterated dimethyl sulfoxide ($D_6$-DMSO) and deuterium oxide ($D_2O$), with methanol as the internal standard.

Example 1

To a 500 ml 3-necked round-bottom flask equipped with a dry ice condenser, thermocouple in glass tube, mechanical stirrer, gas outlet, and dropping funnel was added 116.5g (1.0 mole) of chlorosulfonic acid (99%). A solution of 29.1g (0.5 mole) of acetone dissolved in 75 ml of methylene chloride was added dropwise at such a rate as to maintain the temperature below 20 degrees Centigrade. When the addition was complete, the clear homogeneous solution was warmed to reflux. A vigorous evolution of hydrogen chloride began at about 35 degrees Centigrade with refluxing of the methylene chloride. After 15 minutes reaction white solids began to precipitate, and the reaction continued for about another 3 to 3.5 hours. The white solid was either filtered under a nitrogen blanket and dried under reduced pressure or 109.0g water was added and the 50% solution of product (bottom layer) was isolated.

The calculated yields were in the range of 75-100% of propanone-1,3-disulfonic acid. The proton

3

nuclear magnetic reasonance (H[1]nmr) and carbon 13 (C[13]nmr) analyses indicated the following which were consistent with the structure of propanone-1,3-disulfonic acid:

## H[1] NMR

| Proton Assignment | Chemical Shifts (ppm) D$_2$O Solvent |
|---|---|
| -CH$_2$- | 4.20 |
| HO- | 6.76 |
| CH$_3$-(CH$_3$OH) | 3.30 (Standard) |

## C[13] NMR

| Carbon Assignment | Chemical Shifts (ppm) D$_2$O Solvent |
|---|---|
| -CH$_2$- | 61.42 |
| $-\overset{O}{\overset{\|}{C}}-$ | 194.03 |
| CH$_3$(CH$_3$OH) | 49.00 |

Example 2

Example 1 was repeated under the same conditions of reaction, except no solvent was used and the temperature of the reaction was 14 to 50 degrees Centigrade (1-hour reaction time). The H[1] and C[13]nmr analyses were as follows and were consistent with the structure of propanone-1,3-disulfonic acid:

## H[1] NMR

| | Chemical Shifts (ppm) | |
|---|---|---|
| Proton Assignment | D$_6$-DMSO Solvent | D$_2$O Solvent |
| CH$_2$ | 3.92 | 4.34 |
| OH | 0.61 | 5.19 |

## C[13] NMR

| | Chemical Shifts (ppm) | |
|---|---|---|
| Carbon Assignment | D$_6$-DMSO Solvent | D$_2$O Solvent |
| C=O | 195.82 | 196.18 |
| CH$_2$ | 64.45 | 63.50 |

The elemental analysis of the solid product was calculated as the monohydrate (very hygroscopic) of propanone-1,3-disulfonic acid:

| | Calcd. | Found |
|---|---|---|
| C | 15.25 | 15.6 |
| H | 3.39 | 3.21 |
| S | 27.12 | 28.2 |

4

Example 3

Example 1 was repeated under the same conditions of reaction, except carbon tetrachloride was used as the solvent and the temperature range of reaction was 7 to 52 degrees Centigrade. The elemental analysis of the solid product was as follows:

```
C          15.7
H           2.96
S          28.1
```

Propanone-1,3-disulfonic acid has utility as an alkylation and esterification catalyst, as a solubility enhancer for dimethyl disulfide in concentrated hydrochloric acid solutions, as an electrochemical stripping agent for various metals, as a solvent for various metals by forming water soluble salts some of which are useful transesterification catalysts, as a reactant for forming polymers by undergoing aldol or ketone condensations (e.g., reactions with formaldehyde, glyoxal, phenol-formaldehyde, ketal formation, hydrazine reactions, photochemical condensations, bisulfite reactions, etc.), and as an additive to enhance flame retardancy when using the salts (e.g., ammonium salts).

**Claims**

1. A method for preparing propanone-1,3-disulfonic acid comprising reacting chlorosulfonic acid with acetone.

2. A method according to Claim 1 wherein the reaction is carried out in the presence of a solvent for the reactants.

3. A method according to Claim 2 wherein the solvent is selected form the group consisting of methylene chloride, carbon tetrachloride, 1,1,1-trichloroethane, chloroform, dioxane, acetonitrile, tetrahydrofuran, and ethyl ether.

4. A method according to Claim 1 wherein the reaction is carried out in the absence of a solvent.

5. A method according to Claim 1 wherein the reactants are substantially anhydrous.

6. A method according to Claim 1 wherein the reaction is carried out at a temperature of 4 to 60 degrees Centigrade.

7. A method according to Claim 6 wherein the reaction temperature range is 4 to 40 degrees Centigrade.

8. A method according to Claim 1 wherein the reactants are present in about stoichiometric amounts to a slight stoichiometric excess of chlorosulfonic acid.

9. A method according to Claim 2 wherein the acetone is first dissolved in the solvent and the resulting solution is added to the chlorosulfonic acid.

10. A method according to Claim 9 wherein the reaction mixture is warmed after addition of the acetone to the chlorosulfonic acid until vigorous evolution of hydrogen chloride occurs.

11. A method according to Claim 2 wherein the solvent is refluxed during the reaction.

12. A method according to Claim 1 wherein the reaction is carried out under anhydrous conditions at atmospheric pressure.

13. A method according to Claim 2 wherein the propanone-1,3-disulfonic acid is recovered in an aqueous solution by adding water to dissolve the precipitated product after completion of the reaction, and the aqueous layer is separated from the solvent layer.

**Revendications**

1. Procédé de préparation d'acide propanone-1,3-disulfonique, qui consiste à faire réagir l'acide chlorosulfonique avec l'acétone.

2. Procédé suivant la revendication 1, dans lequel la réaction est conduite en présence d'un solvant pour les corps réactionnels.

3. Procédé suivant la revendication 2, dans lequel le solvant est choisi dans le groupe comprenant le chlorure de méthylène, le tétrachlorure de carbone, le 1,1,1-trichloréthane, le chloroforme, le dioxanne, l'acétonitrile, le tétrahydrofuranne et l'éther éthylique.

4. Procédé suivant la revendication 1, dans lequel la réaction est conduite en l'absence d'un solvant.

5. Procédé suivant la revendication 1, dans lequel les corps réactionnels sont principalement anhydres.

6. Procédé suivant la revendication 1, dans lequel la réaction est conduite à une température de 4 à 60 degrés centigrades.

7. Procédé suivant la revendication 6, dans lequel la plage de températures de réaction va de 4 à 40 degrés centigrades.

8. Procédé suivant la revendication 1, dans lequel les corps réactionnels sont présents en quantités à peu près stoechiométriques jusqu'à un léger excès stoechiométrique d'acide chlorosulfonique.

9. Procédé suivant la revendication 2, dans lequel l'acétone est tout d'abord dissoute dans le solvant et la solution résultante est ajoutée à l'acide chlorosulfonique.

10. Procédé suivant la revendication 9, dans lequel le mélange réactionnel est chauffé après addition de l'acétone à l'acide chlorosulfonique jusqu'à ce qu'un dégagement énergique de chlorure d'hydrogène ait lieu.

11. Procédé suivant la revendication 2, dans lequel on fait refluer le solvant pendant la réaction.

12. Procédé suivant la revendication 1, dans lequel la réaction est conduite dans des conditions anhydres à la pression atmosphérique.

13. Procédé suivant la revendication 2, dans lequel l'acide propanone-1,3-disulfonique est recueilli dans une solution aqueuse par addition d'eau pour dissoudre le produit précipité après la fin de la réaction, et la phase aqueuse est séparée de la phase de solvant.

**Patentansprüche**

1. Verfahren zur Herstellung von Propanon-1,3-disulfonsäure, dadurch **gekennzeichnet,** daß Chlorsulfonsäure mit Aceton umgesetzt wird.

2. Verfahren nach Anspruch 1, dadurch **gekennzeichnet,** daß die Reaktion in Anwesenheit eines Lösungsmittels für die Reaktionsteilnehmer durchgeführt wird.

3. Verfahren nach Anspruch 2, dadurch **gekennzeichnet,** daß das Lösungsmittel aus der Gruppe ausgewählt wird, die besteht aus Methylenchlorid, Tetrachlorkohlenstoff, 1,1,1-Trichlorethan, Chloroform, Dioxan, Acetonitril, Tetrahydrofuran und Ethylether.

4. Verfahren nach Anspruch l, dadurch **gekennzeichnet,** daß die Reaktion in Abwesenheit eines Lösungsmittels durchgeführt wird.

5. Verfahren nach Anspruch 1, dadurch **gekennzeichnet,** daß die Reaktionsteilnehmer im wesentlichen wasserfrei sind.

6. Verfahren nach Anspruch 1, dadurch **gekennzeichnet,** daß die Reaktion bei einer Temperatur von 4 bis 60°C durchgeführt wird.

7. Verfahren nach Anspruch 6, dadurch **gekennzeichnet,** daß der Reaktionstemperaturbereich 4 bis 40°C beträgt.

8. Verfahren nach Anspruch 1, dadurch **gekennzeichnet,** daß die Reaktionsteilnehmer in etwa stöchiometrischen Mengen bis zu einem geringen stöchiometrischen Überschuß, bezogen auf die Chlorsulfonsäure, vorhanden sind.

9. Verfahren nach Anspruch 2, dadurch **gekennzeichnet,** daß das Aceton zuerst in dem Lösungsmittel gelöst wird und daß die entstehende Lösung zu der Chlorsulfonsäure zugegeben wird.

10. Verfahren nach Anspruch 9, dadurch **gekennzeichnet,** daß das Reaktionsgemisch nach der Zugabe des Acetons zu der Chlorsulfonsäure erwärmt wird, bis eine starke Chlorwasserstoffbildung auftritt.

11. Verfahren nach Anspruch 2, dadurch **gekennzeichnet,** daß das Lösungsmittel während der Reaktion am Rückfluß erhitzt wird.

12. Verfahren nach Anspruch 1, dadurch **gekennzeichnet,** daß die Reaktion unter wasserfreien Bedingungen bei Atmosphärendruck durchgeführt wird.

13. Verfahren nach Anspruch 2, dadurch **gekennzeichnet,** daß Propan-1,3-disulfonsäure in einer wäßrigen Lösung wiedergewonnen wird, indem Wasser durch Auflösen des präzipitierten Produktes nach Beendigung der Reaktion zugegeben wird und die wäßrige Schicht von der Lösungsmittelschicht abgetrennt wird.